# EUROPEAN PATENT SPECIFICATION

(11) **EP 0 539 088 B1**
(45) Date of publication and mention of the grant of the patent: **17.07.1996**
(21) Application number: 92309348.8
(22) Date of filing: 14.10.1992
(51) Int. Cl.: C07K 7/56, C07K 1/00, A61K 38/00

(54) **Echinocandin b derivative**
Echinocandin-B-Derivat
Dérivé d'échinocandine B

(30) Priority: 17.10.1991 US 775773
(43) Date of publication of application: 28.04.1993
(73) Proprietor: MERCK & CO. INC., Rahway New Jersey 07065-0900 (US)
(72) Inventor: Bouffard, Frances Aileen, Scotch Plains, NJ 07076 (US); Dropinski, James F., Edison, NJ 08817 (US)
(74) Representative: Thompson, John Dr.

(56) References cited:
- EP-A- 0 447 186
- EP-A- 0 451 957
- EP-A- 0 459 564
- CHEMICAL ABSTRACTS, vol. 114, no. 21, 27 May 1991, Columbus, OH (US); A.A. ADEFARATI et al., p. 441, no. 203279t

## Description

### BACKGROUND OF THE INVENTION

The present invention is directed to a particular antibiotic compound having a superior combination of properties.

Echinocandin B and related fermentation metabolites are known to have antifungal properties when tested in vitro. However, many of the compounds are less effective in vivo and in most cases are further undesirable for therapeutic use because of their lytic activity on human red blood cells. Some derivatives have been prepared in a search to find more useful compounds for human therapeutic use. Most of the derivatives are lipophilic side chain analogs at the α:amino-nitrogen of the hydroxyornithine compound or ethers at the hemiaminal position, the position of the naturally occurring ethers. A number of aminoalkyl ethers were prepared and are the subject of Belgian patent no. 859,067 (1978) and Belgian patent No. 851,310 (1977).

According to the present invention it has been discovered that when the aminoalkyl ether is that derived not from echinocandin B but from a cyclohexapeptide compound in which one of the nuclear amino acids is glutamine instead of threonine, the compound has superior antibiotic activity not only in vitro, but also in vivo. Moreover, the compound is substantially non-lytic toward human blood cells, thereby rendering the compound adaptable for human therapy which has not been possible with many compounds even though they might be active.

The compound of the present invention may be represented by the formula 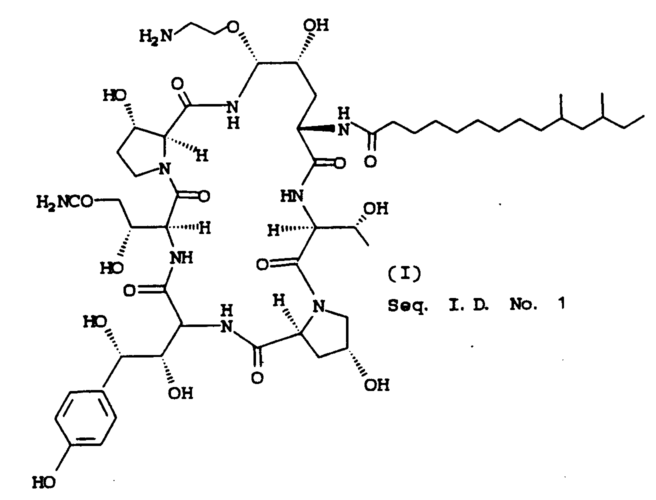
and include acid addition salts thereof.

Pharmaceutically acceptable salts as acid addition salts are those from acids such as hydrochloric, hydrobromic, phosphoric, sulfuric, maleic, citric, acetic, tartaric, succinic, oxalic, malic, glutamic and the like, and include other acids related to the pharmaceutically acceptable salts listed in Journal of Pharmaceutical Science, 66, 2 (1977).

The nucleus of the aminoethyl ether and the starting material are the same since the amino acid of the peptide nucleus is not changed. Thus, both product and starting material have the same Sequence I.D. number.

The compound of the present invention is a white solid having the following spectral characteristics:
¹H NMR Spectra (400 MHz, CD₃OD) δ 1.16(d.3,J=6.2Hz, CH₃-threo),3.12(m,2,OCH₂CH₂NH₂·CF₃COOH), 3.72(m, OCH₂CH₂NH₂·CF₃COOH),4.10(m,1,H4 4,5-(di-OH)-orn), 5.24(d,1,J=2.3Hz, H5 4,5-(di-OH)-orn)
FAB-MS, m/e 1108 (M+1)⁺
The compound is soluble in a variety of organic solvents such as methanol, ethanol, dimethylformamide (DMF), dimethylsulfoxide (DMSO) and the like and also in water.

The antibiotic activity disclosed above is especially of note against fungi causing pathogenic mycotic infections such as Candida albicans, Candida tropicalis and the like. Compound I has been found to significantly prolong the survival of mice infected with Candida albicans and also to eradicate Candida albicans from kidneys of experimentally infected mice. These properties point to a new antifungal drug with great potential in the therapy of human mycotic infections. Additionally, the compound is adapted to be employed for inhibiting or alleviating Pneumocystis carinii infections, prevalent in immune compromised patients and which have usually been fatal.

The compound of the present invention may be obtained by the reaction of 1-[4,5-dihydroxy-N²-(10,12-dimethyl-1-oxotetradecyl)ornithine]-5-(3-hydroxy-glutamine)-6-(3-hydroxyproline)echinocandin B, a natural product with ethanolamine hydrochloride, in the presence of strong acid in an aprotic polar solvent, and thereafter isolated from the reaction mixture, preferably by the use of reverse phase high performance liquid chromatography (HPLC) techniques.

Suitable acids include a strong organic acid or a mineral acid. Examples of strong organic acids are camphorsulfonic acid, p-toluenesulfonic and methanesulfonic acid. Mineral acids include hydrochloric and hydrobromic acid. Hydrochloric and camphorsulfonic acids are preferred.

Suitable solvents include DMSO, DMF, 1-methyl-2-pyrrolidinone, hexamethylphosphoric triamide (HMPA) and the like. Dimethyl sulfoxide is preferred.

The reaction may be carried out by stirring the reactants together at ambient temperature for from one to several days.

The reaction mixture is conveniently purified using HPLC techniques, including utilization of a reverse phase column. The eluants from HPLC are then concentrated and lyophilized as subsequently detailed. The elution is carried out using various concentrations of acetonitrile/water, starting at about 15 percent acetonitrile and then increasing the amount of acetonitrile. The eluting solutions generally contain 0.1 percent trifluoroacetic acid (TFA) or acetic acid and the product on isolation is found in the form of the salt.

The compounds of the present invention are active against many fungi and as previously indicated particularly against Candida species. The antifungal properties may be illustrated with the minimum fungicidal concentration (MFC) determination against certain Candida organisms in a microbroth dilution assay carried out in a Yeast Nitrogen Base (Difco) medium with 1 percent dextrose (YNBD).

In carrying out the assay, Compound I was solubilized in 10 percent dimethyl sulfoxide (DMSO) and diluted to 2560 µg/ml. The compound was then diluted to 256 µg/ml in YNBD. 0.15 mL of the suspension was dispensed to the top row of a 96-well plate (each well containing 0.15 ml of YNBD) resulting in a drug concentration of 128 µg/ml. Two-fold dilutions were then made from the top row to obtain final drug concentrations ranging from 128 to 0.06 µg/ml.

The yeast cultures, maintained,on Sabouraud dextrose agar were transferred to YN broth (Difco) and incubated overnight at 35°C with shaking (250 rpm). After incubation, each culture was diluted in sterile water to yield a final concentration of 1-5 x 10⁶ colony forming units (CFU)/ml.

96-well microplates were inoculated-using a MIC-2000 (Dynatech) which delivers 1.5 ml per well yielding a final inoculum per well of 1.5-7.5 x 10³ cells. The microplates were incubated at 35°C for 24 hours. The minimum inhibitory concentrations (MICs) were recorded as the lowest concentrations of drug showing no visible growth.

After recording the MIC, the plates were shaken to resuspend the cells. Thereafter, 1.5 µl samples from the wells in the 96-well microplate were transferred to a single well tray containing Sabouraud dextrose agar. The inoculated trays were incubated 24 hours at 28°C and then read for minimum fungicidal concentration (MFC). MFC is defined as the lowest concentration of drug showing no growth or less than 4 colonies per spot. The results showed the minimum fungicidal concentration against Candida albicans MY 1055 to be 0.125 µg/ml and against Candida albicans MY 1028 to be 0.25 µg/ml.

Compound I also shows in vivo effectiveness against fungi as seen by the following experiment.

Growth from an overnight SDA culture of Candica albicans MY 1055 was suspended in sterile saline and the cell concentration determined by hemacytometer count and the cell suspension adjusted to 3.75 x 10⁵ cells/ml. Then 0.2 milliliter of this suspension was administered I.V. in the tail vein of mice so that the final inoculum was 7.5 x 10⁴ cells/mouse.

The assay then was carried out by administering aqueous solutions of Compound I at various concentrations intraperitoneally (I.P.), twice daily (b.i.d.) for four consecutive days to 18 to 20 gram female DBA/2 mice, which previously had been infected with Candida albicans (MY 1055) in the manner described above. Distilled water was administered I.P. to C. albicans challenged mice as controls. After seven days, the mice were sacrificed by carbon dioxide gas, paired kidneys were removed aseptically and placed in sterile polyethylene bags containing 5 milliters of sterile saline. The kidneys were homogenized in the bags, serially diluted in sterile saline and aliquots spread on the surface of SDA plates. The plates were incubated at 35°C for 48 hours and yeast colonies were enumerated for determination of colony forming units (CFU) per gram of kidneys. Compound I showed greater than 99 percent reduction of recoverable Candida CFUs at 0.4 mg/kg I.P. twice daily for four consecutive days.

A harmful and potentially fatal side reaction of a number of drugs including certain antibiotically active echinocandin compounds is red blood cell lysis. It was of particular interest when it was found that the compound of this invention exhibited no red blood cell lysis at concentrations far beyond what would be used for therapeutic purposes. The blood cell lysis property may be seen in the determination carried out in the following manner.

The blood employed is a 4 percent suspension of freshly drawn heparinized blood prepared by adding 2 milliliters of blood to 50 milliliters of sterile 5 percent dextrose.

Compound I was solubilized in a small volume of dimethylsulfoxide (DMSO) that was then diluted with distilled water to a final concentration of 5 percent DMSO to obtain a drug suspension of 4.0 mg/ml. A 0.2 milliliter amount of the drug suspension was added to 1.4 milliliters of sterile 5 percent dextrose to obtain the test suspension. A diluent control was also prepared.

A 96-well microliter plate with a well volume of 0.35 ml was used for the assay. Columns 2-12 were filled with 150 µl of sterile 5 percent dextrose. Then, 300 µl of test suspensions were dispensed into the wells in column 1 and serially two-fold diluted in 5 percent dextrose to yield final test concentrations of from 400 to 0.20 µg/ml. 38 µl of red blood cell suspension were added to each well, the plate was gently agitated to mix the well contents and incubated at room temperature for 2 hours, and thereafter observed to determine extent of hemolysis as indicated by complete or partial clearing (lysis).

Minimum lytic concentration (MLC) defined as the lowest concentration of test compound to produce complete or partial lysis of red blood cells was found to be 400 mg/ml.

The compounds of the present invention may also be useful for inhibiting or alleviating Pneumocystis carinii infections in immune compromised patients. The efficacy of the compounds of the present invention for the therapeutic or anti-infective purposes may be demonstrated in studies on immunosuppressed rats in which Sprague-Dawley rats (weighing approximately 250 grams) are immunosuppressed with dexasone in the drinking water (2.0 mg/L) and maintained on a low protein diet for seven weeks to induce the development of pneumocystis pneumonia from a latent infection. Before drug treatment, two rats are sacrificed to confirm the presence of Pneumocystis carinii pneumonia (PCP). Five rats (weighing approximately 150 grams) are injected twice daily for four days subcutaneously (sc) with Compound I in 0.25 ml of vehicle (distilled water). A vehicle control is also carried out. All animals continue to receive dexasone in the drinking water and low protein diet during the treatment period. At the completion of the treatment, all animals are sacrificed, the lungs are removed and processed, and the extent of disease determined by microscopic examination of stained slides for the presence of cysts. The prevention of or reduction of cysts are seen in slides of lungs of treated rats when compared with the number of cysts in lungs of untreated controls or solvent controls.

The outstanding properties are most effectively utilized when the compound is formulated into novel pharmaceutical compositions with a pharmaceutically acceptable carrier according to conventional pharmaceutical compounding techniques.

The novel compositions contain at least a therapeutic antifungal or antipneumocystis amount of the active compound. Generally, the composition contains at least 1% by weight of Compound I or one of the components. Concentrate compositions suitable for dilutions prior to use may contain 90% or more by weight. The compositions include compositions suitable for oral, topical, parenteral (including intraperitoneal, subcutaneous, intramuscular, and intravenous), nasal, and suppository administration, or insufflation. The compositions may be prepacked by intimately mixing Compound I with the components suitable for the medium desired. Compositions formulated for oral administration may be a liquid composition or a solid composition. For liquid preparations, the therapeutic may be formulated with liquid carriers such as water, glycols, oils, alcohols, and the like, and for solid preparations such as capsules and tablets, with solid carriers such as starches, sugars, kaolin, ethyl cellulose, calcium and sodium carbonate, calcium phosphate, koalin, talc, lactose, generally with lubricant such as calcium stearate, together with binders disintegrating agents and the like. Because of their ease in administration, tablets and capsules represent the most advantageous oral dosage form. It is especially advantageous to formulate the compositions in unit dosage form (as hereinafter defined) for ease of administration and uniformity of dosage. Compositions in unit dosage form constitute an aspect of the present invention and for injection take such forms as suspensions, solutions or emulsions in oily or aqueous vehicles such as 0.85 percent sodium chloride or 5 percent dextrose in water and may contain formulating agents such as suspending, stabilizing and /or dispersing agents. Buffering agents as well as additives such as saline or glucose may be added to make the solutions isotonic. The compound also may be solubilized in alcohol/propylene glycol or polyethylene glycol for drip intravenous administration. The compositions also may be presented in unit dosage form in ampoules or in multidose containers, preferably with added preservative. Alternatively, the active ingredients may be in powder form for reconstituting with a suitable vehicle prior to administration.

The term "unit dosage form" as used in the specification and claims refer to physical discrete units, each unit containing a predetermined quantity of active ingredient calculated to produce the desired therapeutic effect in association with the pharmaceutical carrier. Examples of such unit dosage forms are tablets, capsules, pills, powder packets, wafers, measured units in ampoules or in multidose containers and the like. A unit dosage of the present invention will generally contain from 100 to 200 milligrams of one of the compounds.

When the compound is for antifungal use any method of administration may be employed. For treating mycotic infections, oral administration is frequently preferred.

When the compound is to be employed for control of pneumocystis infections, it is desirable to directly treat lung and bronchi. For this reason inhalation methods are preferred. For administration by inhalation, the compounds of the present invention are conveniently delivered in the form of an aerosol spray presentation from pressurized packs or nebulisers. The preferred delivery system for inhalation is a metered dose inhalation (MDI) aerosol which may be formulated as a suspension of Compound I in a suitable propellant such as fluorocarbon or hydrocarbons.

The following examples illustrate the preparation of Compound I and compositions of Compound I useful in the therapeutic application of Compound I, but are not to be construed as limiting.

### EXAMPLE I

A solution of 200 milligrams (0.19 mmol) of 1-[4,5-dihydroxy-N²-(10,12-dimethyl-1-oxotetradecyl)-ornithine]-5-(3-hydroxyglutamine)-6-(3-hydroxyproline) echinocandin B (Seq. I.D. No. 1), 1.83 grams of ethanolamine hydrochloride (19 mmol), and 44 milligrams (0.19 mmol) of (1S)-(+)-10-camphorsulfonic acid in 8 milliliters of anhydrous dimethylsulfoxide was stirred at 25°C for a period of 4 days. The reaction mixture was diluted with 16 milliliters of water and flash chromatographed on a "LICHROPREP" (E. Merck) RP-18(40-63µm,8 g) column, packed in 15 percent acetonitrile/water. The column was then eluted first with 15 percent acetonitrile/water (2 x 100 mL) then with 35 percent acetonitrile/water. The fractions from the latter elution were combined, concentrated and lyophilized to obtain 65 milligrams of product. The latter was purified by preparative HPLC ["ZORBAX" (DuPont) C18, 21.2 x 250 mm, 40 percent acetonitrile/water (0.1% CF₃COOH)], and the eluates concentrated and lyophilized to obtain 1-[4-hydroxy-5-aminoethyloxy-N²-(10,12-dimethyl-1-oxotetradecyl)-ornithine]-5-(3-hydroxyglutamine)-6-(3-hydroxyproline)-echinocandin B trifluoroacetate. (Seq. I.D. No. 1) The product had the spectral characteristics previously set forth.

### EXAMPLE II

1000 hard gelatin capsules, each containing 500 mg of Compound I are prepared from the following formulation:

| Compound | Grams |
|---|---|
| Compound I (Seq. I.D. No. 1) | 500 |
| Starch | 250 |
| Lactose | 750 |
| Talc | 250 |
| Calcium stearate | 10 |

A uniform mixture of the ingredients is prepared by blending and used to fill two-piece hard gelatin capsules.

### EXAMPLE III

An aerosol composition may be prepared having the following formulation:

| | Per Canister |
|---|---|
| Compound I (Seq. I.D. No. 1) | 24 mg |
| Lecithin NF Liquid Concentrated | 1.2 mg |
| Trichlorofluoromethane, NF | 4.026 g |
| Dichlorodifluoromethane, NF | 12.15 g |

### EXAMPLE IV

1000 compressed tablets each containing 500 mg of Compound I are prepared from the following formulation:

| Compound | Grams |
|---|---|
| Compound I (Seq. I.D. No. 1) | 500 |
| Starch | 750 |
| Dibasic calcium phosphate, hydrous | 5000 |
| Calcium stearate | 2.5 |

The finely powdered ingredients are mixed well and granulated with 10 percent starch paste. The granulation is dried and compressed into tablets.

### PREPARARION OF STARTING MATERIAL

The starting material 1-[4,5-dihydroxy-N²-(10,12-dimethyl-1-oxotetradecyl)ornithine]-5-(3-hydroxyglutamine)-6-(3-hydroxyproline)echinocandin B may be obtained by cultivating Z. arboricola ATCC 20868 in a nutrient medium enriched in mannitol as the primary source of carbon as described in U.S. Patent no. 5,021,341, June 4, 1991.

## Claims

1. 1-[4-hydroxy-5-aminoethyloxy-N²-(10,12-dimethyl-1-oxotetradecyl)ornithine]-5-(3-hydroxyglutamine)-6-(3-hydroxyproline)echinocandin B having the formula

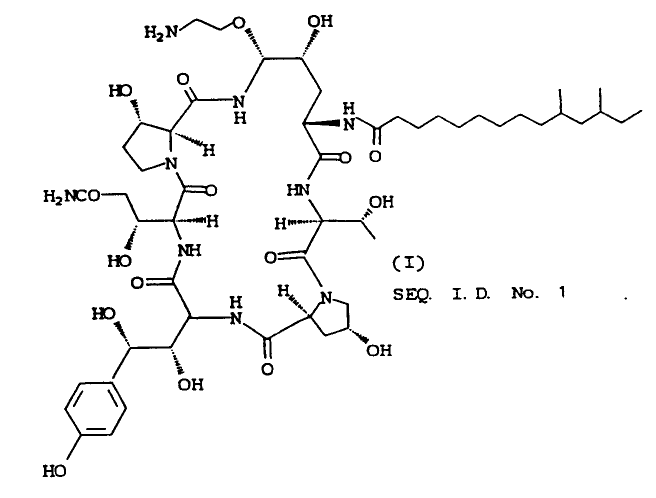

2. An antibiotic composition comprising a therapeutic amount of the compound of Claim 1 in a pharmaceutically acceptable carrier.

3. A composition according to Claim 2 in unit dosage form wherein the compound of Claim 1 is present in an amount of 10 milligrams to 200 milligrams.

4. The use of a compound of Claim 1 for the manufacture of a medicament for treating mycotic infections.

5. A process for the preparation of the compound as claimed in Claim 1 which comprises reacting 1-[4,5-dihydroxy-N²-(10,12-dimethyl-1-oxotetradecyl)ornithine]-5-(3-hydroxyglutamine)-6-(3-hydroxyproline)echinocandin B with ethanolamine hydrochloride, in the presence of strong acid in an aprotic polar solvent.

6. A process for the preparation of a pharmaceutical composition which comprises mixing the compound as claimed in Claim 1 or a pharmaceutically acceptable acid addition salt thereof with a pharmaceutically acceptable carrier.

## Patentansprüche

1. 1-[4-Hydroxy-5-aminoethyloxy-N²-(10,12-dimethyl-1-oxotetradecyl)ornithin]-5-(3-hydroxyglutamin)-6-(3-hydroxyprolin)echinocandin B mit der Formel

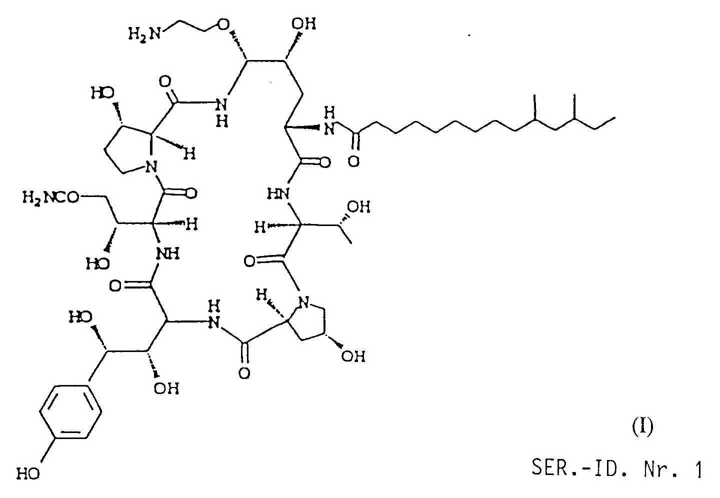

2. Eine Antibiotikumzusammensetzung, umfassend eine therapeutische Menge der Verbindung nach Anspruch 1 in einem pharmazeutisch verträglichen Träger.

3. Eine Zusammensetzung nach Anspruch 2 in Einheitsdosierungsform, wobei die Verbindung nach Anspruch 1 in einer Menge von 10 Milligramm bis 200 Milligramm vorliegt.

4. Die Verwendung einer Verbindung nach Anspruch 1 für die Herstellung eines Medikamentes zur Behandlung mykotischer Infektionen.

5. Ein Verfahren für die Herstellung der wie in Anspruch 1 beanspruchten Verbindung, umfassend Umsetzen von 1-[4,5-Dihydroxy-N²-(10,12-dimethyl-1-oxotetradecyl)ornithin]-5-(3-hydroxyglutamin)-6-(3-hydroxyprolin)echinocandin B mit Ethanolaminhydrochlorid in der Anwesenheit einer starken Säure in einem aprotischen polaren Lösungsmittel.

6. Ein Verfahren für die Herstellung einer pharmazeutischen Zusammensetzung, umfassend Mischen der wie in Anspruch 1 beanspruchten Verbindung oder eines pharmazeutisch verträglichen Säureadditionssalzes davon mit einem pharmazeutisch verträglichen Träger.

## Revendications

1. La 1-[4-hydroxy-5-aminoéthyloxy-N²-(10,12-diméthyl-1-oxotetradécyl)ornithine]-5-(3-hydroxy-glutamine)-6-(3-hydroxyproline)échinocandine B représenté par la formule

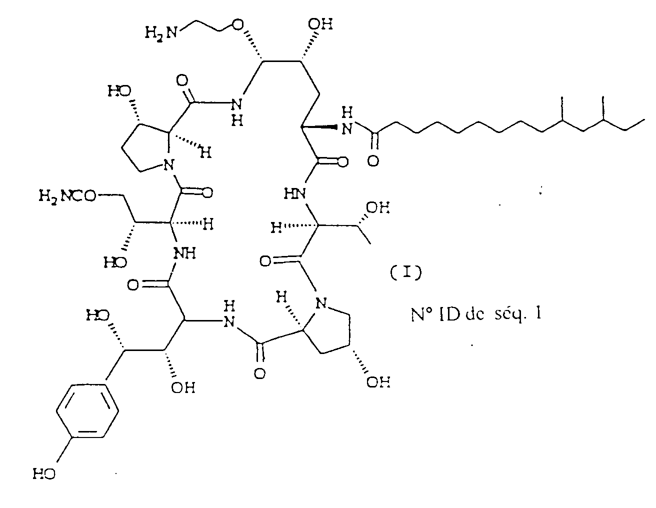

2. Composition antibiotique comprenant une quantité thérapeutique du composé selon la revendication 1 dans un vecteur pharmiceutiquement acceptable.

3. Composition selon la revendication 2 sous forme de doses unitaires dans lesquelles le composé selon la revendication 1 est présent en quantité de 10 milligrammes à 200 milligrammes.

4. Utilisation du composé selon la revendication 1 pour la fabrication d'un médicament pour le traitement des mycoses.

5. Procédé de préparation d'un composé selon la revendication 1 comprenant la réaction de la 1-[4,5-dihydroxy-N²-(10,12-diméthyl-1-oxotetradécyl-ornithine]-5-(3-hydroxy-glutamine)-6-(3-hydroxyproline)échinocandine B avec le chlorhydrate d'éthanolamine en présence d'un acide fort dans un solvant polaire aprotique.

6. Procédé de préparation d'une composition pharmaceutique comprenant le mélange du composé selon la revendication 1 ou d'un sel d'addition d'acides pharmaceutiquement acceptable de ce composé avec un vecteur pharmaceutiquement acceptable.


SEQUENCE LISTING
INFORMATION FOR SEQ ID NO: 1
(i) SEQUENCE CHARACTERISTICS:
(A) LENGTH: 6
(B) TYPE: AMINO ACID
(C) STRANDEDNESS: NA
(D) TOPOLOGY: CIRCULAR
(ii) MOLECULE TYPE:
(A) DESCRIPTION: PEPTIDE
(xi) SEQUENCE DESCRIPTION: SEQ ID NO: 1
Xaa Thr Xaa Xaa Xaa Xaa
1               5